# EUROPEAN PATENT APPLICATION

(11) **EP 1 657 232 A1**
(43) Date of publication of application: **17.05.2006**
(21) Application number: 04026256.0
(22) Date of filing: 05.11.2004
(51) Int. Cl.: C07C 57/30, C07C 57/58, A61K 31/192

(54) **Use of S-enantiomers of alpha-sustituted aryl acetic acids for the prevention of Alzheimer's disease**

(71) Applicant: CELLZOME AG, 69117 Heidelberg (DE)
(72) Inventor: Ramsden, Nigel, Royston, Herts SG8 7QP (GB); Wilson, Francis, Herts. AL 7 4QJ (GB)
(74) Representative: Hörschler, Wolfram Johannes

(57) **Abstract**

The present invention relates to a novel class of y-secretase inhibitors, including pharmaceutically acceptable salts thereof, which are useful as therapeutic compounds, particularly in the treatment of Alzheimer's disease. The present invention also relates to a process for preparing said compounds as well as a process for the identification of such compounds.

## Description

The present invention relates to a novel class of γ-secretase modulators, including pharmaceutically acceptable salts thereof, which are useful as therapeutic compounds, particularly in the treatment of Alzheimer's disease. The present invention also relates to a process for the identification of such compounds.

Alzheimer's disease is the most common form of age-related neurodegenerative illness.

It is primarily, but not exclusively associated with aging and presents clinically not only by progressive loss of memory, cognition, reasoning, judgement, but also emotional instability and gradually leads to profound mental deterioration and death.

The defining pathological hallmarks of Alzheimer's disease are the presence of neurofibrillar tangles and amyloid plaques in the brain, which are also thought to play a central role in the pathogenesis of the disease.

These plaques mainly consist of peptides formed as cleavage products of the β-amyloid precursor protein (APP), a 695 amino acid protein, whose function so far has only been the subject of various hypotheses.

APP is processed in two steps, a first step (catalyzed by β-secretase) giving rise to a secreted peptide and the membrane-bound C99-fragment.

C99 is a substrate for a second proteolytic activity called γ-secretase resulting, inter alia, in the production of peptides in the range of 37-42 residues.

The longer isoform, Aβ42, is selectively increased in patients carrying certain mutations in a particular protein (presenilin) which have been correlated with early-onset familial Alzheimer's disease.

Therefore, Aβ42 is believed by many to be the main culprit of the pathogenesis of Alzheimer's disease.

It has now become clear that the γ-secretase activity cannot be ascribed to a single particular protein, but is in fact associated with an assembly of different proteins comprising Aph1, Nicastrin, Presenilin and Pen-2 (reviewed by De Strooper (2003) Neuron 38, 9)

Thus, although the molecular mechanism of the cleavage-step has remained elusive until present, the γ-secretase-complex has become one of the prime targets in the search for compounds for the treatment of Alzheimer's disease.

Other hints in the search for novel treatments came from epidemiological studies, an example being the finding that the uptake of certain non-steroidal anti-inflammatory drugs ("NSAID") seems to correlate with a reduced risk of developing Alzheimer's disease (Akiyama et al (2000), Neurobiol. Aging 21, 383; McGeer et al (1996), Neurology 47: 425; Rogers et al (1993), Neurology 43, 1609; Anthony et al, Neurology 54, 2066; Stewart t al (1997), Neurology 48, 626; In't Veld et al (1999), Neurobiol Aging 19, 607)

Indeed, this finding was recently supported by biochemical studies in which an effect of certain NSAIDs on γ-secretase was shown (Weggen et al (2001) Nature 414(6860):212-6; Morihara et al (2002) J Neurochem. (4):1009-12).

Important NSAIDs are 2-aryl acetic and propionic ("2-methyl acetic") acids, such as ibuprofen or flurbiprofen.

Furthermore it has been suggested that it would be advantageous to identify an enantiomerically stable R-NSAID (Eriksen (2003) J. Clin. Invest. 112(3):440-9).

However, if the NSAID template can be modified to remove cyclo-oxygenase activity without losing γ-secretase activity, then it would be advantageous to use the S-enantiomer (Caldwell et al. (1988), Biochem. Pharmacol. 37 105-114).

Thus, the object of the present invention is to provide a new class of compounds as γ-secretase modulators, which have a structural similarity to S-enantiomeric aryl acetic acid NSAIDs.

The object can be achieved by aryl acetic acid γ-secretase modulator compounds having a chemical structure comprising the substructure of formula (Ia) 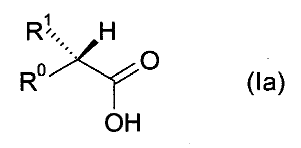 or a pharmaceutically acceptable salt or ester thereof, wherein

R⁰ is aryl and R¹ is other than H, COOH or R⁰, provided that at least one of R⁰, R¹ is selected to prevent inhibition of cyclooxygenase activity by the compound.

Surprisingly, it is found that the compounds of the present invention having a structural similarity to S-enantiomeric aryl acetic acid NSAIDs may have no cyclooxygenase inhibitory activity when in α position to the carboxylic acid group an R¹ group is introduced having a higher spatial demand compared to the methyl and hydrogen group in that position and/or R° can be chosen to prevent cyclooxygenase inhibitory activity.

It is further found that the compounds of the present invention may have a higher stability towards epimerization because the epimerizing enzyme presumably cannot recognize the absolute configuration as outlined in formula (Ia). Thus, the compounds of the present invention are preferably substantially free of their counter-enantiomers.

Within the meaning of the present invention the term "substantially free of the counter-enantiomer" means that the enantiomeric excess (ee) is at least 90%, more preferred at least 99% and most preferred at least 99,9%.

Within the meaning of the present invention the terms are used as follows:
"Alkyl" means a straight-chain or branched carbon chain.
"C₁₋₄ alkyl" means an alkyl chain having 1 - 4 carbon atoms, e.g. at the end of a molecule methyl, ethyl, n-propyl, isopropyl, n-butyl, isobutyl, sec-butyl, tert-butyl or amidst, e.g. - CH₂-, -CH₂-CH₂-, -CH(CH₃)-, -C(CH₂)-, -CH₂-CH₂-CH₂-, -CH(C₂H₅)-, -CH(CH₃)₂-.
"C₂₋₄ alkyl" means C₁₋₄ alkyl other than methyl and -CH₂-.
"C₁₋₆ alkyl" means an alkyl chain having 1 - 6 carbon atoms, e.g. C₁₋₄ alkyl, methyl, ethyl, n-propyl, isopropyl, n-butyl, isobutyl, sec-butyl; tert-butyl, n-pentane, n-hexane, or amidst, e.g. -CH₂-, -CH₂-CH₂-, -CH(CH₃)-, -C(CH₂)-, -CH₂-CH₂-CH₂-, -CH(C₂H₅)-, -CH(CH₃)₂-. Each hydrogen of a C₁₋₆ alkyl carbon may be replaced by a substituent.
"C₁₋₁₀ alkyl" means an alkyl chain having 1 to 10 carbon atoms, e.g. C₁₋₆ alkyl, C₁₋₄ alkyl, methyl, ethyl, n-propyl, isopropyl, n-butyl, isobutyl, sec-butyl; tert-butyl, n-pentane, n-hexane, heptane octane, nonane, decane, or amidst, e.g. -CH₂-, -CH₂-CH₂-, -CH(CH₃)-, - C(CH₂)-, -CH₂-CH₂-CH₂-, -CH(C₂H₅)-, -CH(CH₃)₂-. Each hydrogen of a C₁₋₁₀ alkyl carbon may be replaced by a substituent.
"C₂₋₄ alkenyl" means an unsaturated alkyl group having at least one double bond and having 2 -4 carbon atoms, e.g. -CH=CH₂, -CH=CH-CH₃, -CH₂-CH=CH₂, -CH=CH-CH₂₋CH₃, -CH=CH-CH=CH₂, or amidst, e.g. -CH=CH-. Each hydrogen of a C₂₋₄ alkenyl carbon may be replaced by a substituent.
"C₂₋₁₀ alkenyl" means an unsaturated alkyl group having at least one double bond and having 2 - 10 carbon atoms, e.g. C₂₋₄ alkenyl, . -CH=CH₂, -CH=CH-CH₃, -CH₂-CH=CH₂, - CH=CH-CH₂-CH₃, -CH=CH-CH=CH₂, -(CH₂)₃-CH=CH₂, or amidst, e.g. -CH=CH-. Each hydrogen of a C₂₋₁₀ alkenyl carbon may be replaced by a substituent.
"C₃₋₁₀ alkynyl" means an unsaturated alkyl group having at least one triple bond and having 3 - 10 carbon atoms, e.g. -CH₂-C≡CH. Each hydrogen of a C₃₋₁₀ alkenyl carbon may be replaced by a substituent.
"C₃₋₇ cycloalkyl" or "C₃₋₇ cycloalkyl ring" means a cyclic alkyl chain having 3 - 7 carbon atoms, e.g. cyclopropyl, cyclobutyl, cyclopentyl, cyclohexyl, cyclohexenyl, cycloheptyl. Each hydrogen of a cycloalkyl carbon may be replaced by a substituent.
"Halogen" means fluoro, chloro, bromo or iodo. It is generally preferred that halogen is fluoro or chloro. "Heterocyclyl" or "heterocycle" means a cyclopentane, cyclohexane or cycloheptane ring that may contain up to the maximum number of double bonds (aromatic or non-aromatic ring which is fully, partially or un-saturated) wherein at least one carbon atom up to 4 carbon atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for a heterocycle are furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine; imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, azepine or homopiperazine. "Heterocycle" means also azetidine.
"Heterobicyclyl" or "heterobicycle" means a heterocycle which is condensed with phenyl, C₃₋₇ cycloalkyl or an additional heterocycle to form a bicyclic ring system. "Condensed" to form a bicyclic ring means that two rings are attached to each other by sharing two ring atoms. Examples for a heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, quinoline, dihydroquinoline, tetrahydroquinoline, decahydroquinoline, isoquinoline, decahydroisoquinoline, tetrahydroisoquinoline, dihydroisoquinoline, benzazepine, purine or pteridine.
"Aryl" means an aromatic residue being substituted or unsubstituted and having at least one aromatic ring moiety. This aromatic ring may contain heteroatoms and may be fused or unfused. Preferred "aryl" residues are phenyl, naphthyl, heterocycles containing at least one aromatic ring ("heteroaryl") and heterobicycles containing at least one aromatic ring ("heterobiaryl").

Preferred compounds of formulae (I), (Ia) and (Ib) are those compounds in which one or more of the residues contained therein have the meanings given below, with all combinations of preferred substituent definitions being a subject of the present invention. With respect to all preferred compounds of the formulae (I), (Ia) and (Ib) the present invention also includes all tautomeric and stereoisomeric forms and mixtures thereof in all ratios, and their pharmaceutically acceptable salts or esters.

In preferred embodiments of the present invention, the substituents of the formulae (I), (Ia) and (Ib) independently have the following meaning. Hence, one or more of the substituents can have the preferred or more preferred meanings given below.

A preferred compound has the formula (I) 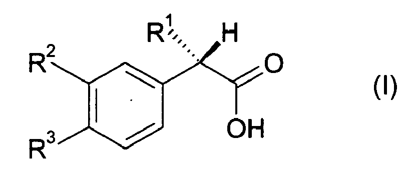 or a pharmaceutically acceptable salt or ester thereof, wherein R¹ is other than H, COOH or 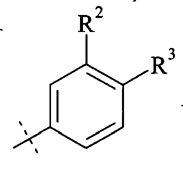 and at least one of R¹, R², R³ is selected to prevent inhibition of cyclooxygenase activity by the compound of formula (I).

Preferably, R², R³ are independently selected from the group consisting of H; halogen; C₁₋₄ alkyl; C₂₋₄ alkenyl; O-C₁₋₄-alkyl; and R⁴-Q; wherein each C₁₋₄ alkyl and C₂₋₄ alkenyl is optionally substituted with one or more halogen, which are the same or different; provided that at least one of R², R³ includes a cyclic moiety; Optionally, R², R³ form together with the carbon atoms to which they are attached to a ring R⁵;
Q is a covalent bond; -C(O)-; -O-; -CH₂-O-; -O-CH₂-; -NH-; -CH₂-NH-; -NH-CH₂-
R⁴, R⁵ are independently selected from the group consisting of phenyl; heterocyclyl and heterobicyclyl and optionally substituted with one or more R⁶ independently selected from the group consisting of halogen; C₁₋₄ alkyl; O-C₂₋₄ alkenyl; phenyl; heterocyclyl; heterobicyclyl; OH; and oxo (=O), where the ring is at least partially saturated; wherein each C₁₋₄ alkyl; C₂₋₄ alkenyl; phenyl; heterocyclyl and heterobicyclyl is optionally substituted with one or more halogen, which are the same or different.

Preferably, R¹ is C₂₋₄ alkyl; C₂₋₄ alkenyl; O-C₁₋₄-alkyl; and R^{4a}-Q^{a}-; wherein each C₂₋₄ alkyl and C₂₋₄ alkenyl is optionally substituted with one or more halogen, which are the same or different and wherein Q^{a} is a covalent bond; -C(O)-; -CH₂-O- or -O-CH₂-; and R^{4a} is phenyl; heterocyclyl or heterobicyclyl and optionally substituted with one or more R^{6a} independently selected from the group consisting of halogen; C₁₋₄ alkyl; O-C₂₋₄ alkenyl; OH; oxo (=O), where the ring is at least partially saturated; wherein each C₁₋₄ alkyl and C₂₋₄ alkenyl is optionally substituted with one or more halogen, which are the same or different.

More preferred, one of R², R³ is H or F and the other is R⁴-Q.

Preferably, R⁴ is phenyl; thiophenyl; 2,5-dihydropyrrolyl; or isoindolin-2one-3-yl.

Preferably, R⁵ is phenyl; indolyl; or oxazolyl.

Preferably, R⁶ is H; CH₃; CF₃; OCF₃; F; Cl; Br or I.

The term "substituted" as used herein includes both part and full substitution. Substituents can be either saturated or unsaturated.

Pharmaceutically acceptable esters are those according to formulae (I), (Ia) and (Ib) in which H of the carboxyl group is replaced by an organic residue. Suitable organic residues are known to a person skilled in the art. Preferred residues include the following:

An unsubstituted or at least monosubstituted alkyl, preferably a C₁₋₁₀ alkyl, an alkenyl, preferably C₂₋₁₀ alkenyl, an alkynyl, preferably C₃₋₁₀ alkynyl, and an unsubstituted or at least monosubstituted ring, either saturated or unsaturated, having 3 to 6 C-atoms, and which may contain in the ring one or more heteroatoms from the group N, S or O, and which heteroatom may be identical or different if more than one heteroatom is present.

Said substituents being selected from the group consisting of halogen, alkyl, alkenyl, alkynyl, N, S, O, carboxyl, sulphonyl, and the like and which can be further substituted.

Described below are exemplary pharmaceutically acceptable salts of the compounds according to the invention which are included herein. The list of the different salts stated below is not meant to be complete and limiting.

Compounds according to the invention which contain one or more acidic groups can be used according to the invention, e.g. as alkali metal salts, alkaline earth metal salts or as ammonium salts. More precise examples of such salts include sodium salts, potassium salts, calcium salts, magnesium salts or salts with ammonia or organic amines such as, e.g. ethylamine, ethanolamine, triethanolamine or amino acids.

Compounds according to the invention which contain several acidic groups can simultaneously form different salts.

Compounds according to the invention which contain one or more basic groups, i.e. groups which can be protonated, can be present and can be used according to the invention in the form of their addition salts with inorganic or organic acids.

Examples for suitable salts include hydrogen chloride, hydrogen bromide, phosphoric acid, sulfuric acid, nitric acid, methanesulfonic acid, p-toluenesulfonic acid, napthalenedisulfonic acid, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfamic acid, phenylpropionic acid, gluconic acid, ascorbic acid, isonicotinic acid, citric acid, adipic acid and other acids known to a person skilled in the art.

The term "pharmaceutically acceptable" means approved by a regulatory agency such as the EMEA (Europe) and/or the FDA (US) and/or any other national regulatory agency for use in animals, preferably in humans.

Compounds according to the invention which contain several basic groups can simultaneously form different salts.

If a compound according to the invention simultaneously contains acidic and basic groups in the molecule, the invention also includes, in addition to the salt forms mentioned, inner salts or betaines.

The respective salts of the compounds according to the invention can be obtained by customary methods which are known to the person skilled in the art, for example by contacting these with an organic or inorganic acid or base in a solvent or dispersant, or by anion exchange or cation exchange with other salts.

Furthermore, the invention includes all salts of the compounds according to the invention which, owing to low physiological compatibility, are not directly suitable for use in pharmaceuticals but which can be used, for example, as intermediates for chemical reactions or for the preparation of pharmaceutically acceptable salts or which might be suitable for studying γ-secretase modulating activity of a compound according of the invention in any suitable manner, such as any suitable in vitro assay.

The present invention furthermore includes all solvates of the compounds according to the invention.

The present invention furthermore includes derivatives/prodrugs (including the salts thereof) of the compounds according to the invention which contain physiologically tolerable ad cleavable groups and which are metabolized in animals, preferably mammals, most preferably humans into a compound according to the invention.

The present invention furthermore includes the metabolites of the compounds according to the invention.

The term "metabolites" refers to all molecules derived from any of the compounds according to the invention in a cell or organism, preferably mammal.

Preferably the term relates to molecules which differ from any molecule which is present in any such cell or organism under physiological conditions

The structure of the metabolites of the compounds according to the invention will be obvious to any person skilled in the art, using the various appropriate methods

The compounds according to general formula (I) can be prepared according to methods published in the literature or, respectively, analogous methods.

Methods for synthesis of the compounds are described e.g., in Houben-Weyl, Methoden der Organischen Chemie (Methods of Organic Chemistry), Thieme-Verlag, Stuttgart, or Organic Reactions, John Wiley & Sons, New York.

Depending on the circumstances of the individual case, in order to avoid side reactions during the synthesis of a compound of the general formula (I), it can be necessary or advantageous to temporarily block functional groups by introducing protective groups and to deprotect them in a later stage of the synthesis, or introduce functional groups in the form of precursor groups which in a later stage are converted into the desired functional groups. Such synthesis strategies and protective groups and precursor groups which are suitable in an individual case are known to the person skilled in the art.

If desired, the compounds of the formula (I) can be purified by customary purification procedures, for example by recrystallization or chromatography. The starting compounds for the preparation of the compounds of the formula (I) are commercially available or can be prepared according to or analogously to literature procedures.

Another aspect of the invention is a pharmaceutical composition comprising a compound or a pharmaceutically acceptable salt thereof according to the present invention together with a pharmaceutically acceptable carrier. Yet another aspect of the present invention is a pharmaceutical composition, comprising one or more additional compounds or pharmaceutically acceptable salts thereof selected from the group consisting of compounds of the present invention and not being the first compound; other pharmaceutically active compounds suitable to treat or prevent Alzheimer's disease; and other γ-secretase modulators.

Yet another aspect of the present invention is a compound or a pharmaceutically acceptable salt thereof according to the present invention for use as a medicament.
a. Alzheimer's disease;
b. One or more diseases associated with the modulation of γ-secretase activity;
c. One or more diseases associated with an elevated level of Aβ42-production.

Another aspect of the present invention is the use of a compound according to the present invention for the modulation of γ-secretase activity.

As used herein, the term "modulation of γ-secretase activity" refers to an effect on the processing of APP by the γ-secretase-complex. Preferably it refers to an effect in which the overall rate of processing of APP remains essentially as without the application of said compounds, but in which the relative quantities of the processed products are changed, more preferably in such a way that the amount of the Aβ42-peptide produced is reduced.

It has been previously shown, that the γ-secretase complex is also involved in the processing of the Notch-protein (e.g. reviewed in Schweisguth F (2004), Curr Biol 14: R129-38).

Notch is a signaling protein which plays a crucial role in developmental processes (e.g. reviewed in Schweisguth F (2004), Curr Biol 14: R129-38).

Particularly with respect to the use of said compounds for the modulation of γ-secretase activity in therapy, it seems advantageous not to interfere with the Notch-processing activity of the γ-secretase activity in order to avoid putative undesired side-effects.

Thus, compounds are preferred which do not show an effect on the Notch-processing activity of the γ-secretase-complex.

Within the meaning of the invention, "effect on the Notch processing activity" includes both an inhibition or an activiation of the Notch-processing activity by a certain factor.

A compound is defined as not having an effect on the Notch processing activity, if said factor is smaller than 20, preferably smaller than 10, more preferably smaller than 5, most preferably smaller than 2 in the respective assay as described in Shimizu et al (2000). Mol. Cell. Biol; Vol. 20: 6913-6922 at a concentration of 30 µM.

Such a modulation can be carried out, e.g. in animals such as mammals. Exemplary mammals are mice, rats, guinea pigs, monkeys, dogs, cats. The modulation can also be carried out in humans.

In a particular embodiment of the invention, said modulation is performed in vitro or in cell culture.

As known to the person skilled in the art, several in vitro and cell culture assays are available.

An example for such an assay is described in WO-03/008635.

Concentrations of the various products of the cleavage (the Aβ-peptides) can be determined by various methods known to a person skilled in the art. Examples for such methods include determination of the peptides by mass-spectrometry or detection by antibodies.

Suitable antibodies are available for example from The Genetics Company, Inc., Switzerland.

Further information are disclosed for example in N. Ida et al. (1996), J. Biological Chemistry; Vol. 271: No.37, 22908 - 22914, and M. Jensen et al. (2000), Mol.Med. (6): 291-302. Antibody-based kits are also available from Innogenetics, Belgium.

Cells which can be employed in such assays include cells which physiologically express the γ-secretase complex and cells which transiently or stably express some or all interactors of the γ-secretase complex.

Numerous available cell lines suitable for such assays are known to the skilled person.

Cells and cell lines of neuronal or glial origin are particularly suitable. Furthermore, cells and tissues of the brain as well as homogenates and membrane preparations thereof may be used.

Such assays might be carried out for example, to study the effect of the compounds according to the invention in different experimental conditions and configurations.

Furthermore, such assays might be carried out as part of functional studies on the γ-secretase complex.

For example, either one or more interactors (either in their wild-type form or carrying certain mutations and/or modifications) of the γ-secretase complex of an animal, preferably a mammal, more preferably humans, might be expressed in certain cell lines and the effect of the compounds according to the invention might be studied.

Mutated forms of the interactor(s) used can either be mutated forms which have been described in certain animals, preferably mammals, more preferably humans or mutated forms which have not previously been described in said animals.

Modifications of the interactors of the γ-secretase complex include both any physiological modification of said interactors and other modifications which have been described as modifications of proteins in a biological system.

Examples of such modifications include, but are not limited to glycosylation, phosphorylation, prenylation, myristylation, farnesylation.

The activity of the γ-secretase can be modulated in different ways, i.e. resulting in different profiles of the various Aβ-peptide.

Uses of a compound for the preparation of a medicament for the modulation of γ-secretase activity resulting in a decrease in the relative amount of produced Aβ42-protein are preferred.

Respective dosages, routes of administration, formulations etc are disclosed further below.

The invention further relates to the use of the compounds according to the invention for the preparation of a medicament for the treatment of a disease associated with an elevated level of Aβ42-production.

As used herein, the term "treatment" is intended to refer to all processes, wherein there may be a slowing, interrupting, arresting, or stopping of the progression of a disease, but does not necessarily indicate a total elimination of all symptoms.

As used herein, the term "elevated level of Aβ42-production" refers to a condition in which the rate of production of Aβ42-peptide is increased due to an overall increase in the processing of APP or, preferably, it refers to a condition in which the production of the Aβ42 peptide is increased due to a modification of the APP-processing profile in comparison to the wild-type/non-pathological situation.

As outlined above, such an elevated Aβ42-level is a hallmark of patients developing or suffering from Alzheimer's disease.

Furthermore the invention relates to a composition comprising a compound according to the invention in a mixture with an inert carrier.

In a preferred embodiment, the invention relates to a composition comprising a compound according to the invention in a mixture with an inert carrier, where said inert carrier is a pharmaceutical carrier.

The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the therapeutic, preferably in purified form, together with a suitable amount of carrier so as to provide the form for proper administration to the patient. The formulation should suit the mode of administration.

Compound according to the present invention may be prepared in analogy to the synthesis of known NSAID (see e.g. US-A 6,160,018). If compounds are synthesised as their racemate, standard methods well known to a person skilled in the art can be used to separate the racemates in order to obtain the enantiomers.

A preparation of preferred compounds according to the present invention comprises the steps of coupling phenyl acetic acid derivative, optionally said derivative being protected, with an appropriate aromatic compound and optional further functionalisation and deprotection of the thus-obtained biphenyl compound.

The compounds according to the invention and their pharmaceutically acceptable salts, optionally in combination with other pharmaceutically active compounds suitable to treat or prevent Alzheimer's disease such as Aricept (Eisai), Donepezil (Pfizer), Cognex (Warner-Lambert), Tacrine (Warner-Lambert), Axura (Merz), Memantine (Merz) or with any other of the drugs known to a person skilled in the art suitable to treat or prevent Alzheimer's disease, can be administered to animals, preferably to mammals, and in particular humans, as pharmaceuticals by themselves, in mixtures with one anther or in the form of pharmaceutical preparations.

Various delivery systems are known and can be used to administer a compound of the invention for the treatment of Alzheimer's disease/for the modulation of the γ-secretase activity, e.g., encapsulation in liposomes, microparticles, and microcapsules:

If not delivered directly to the central nervous system, preferably the brain, it is advantageous to select and or modify methods of administration in such a way as to allow the pharmaceutical compound to cross the blood-brain barrier.

Methods of introduction include but are not limited to intradermal, intramuscular, intraperitoneal, intravenous, subcutaneous, intranasal, epidural, and oral routes.

The compounds may be administered by any convenient route, for example by infusion, by bolus injection, by absorption through epithelial or mucocutaneous linings and may be administered together with other biologically active agents.

Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In another embodiment, the Therapeutic can be delivered in a vesicle, in particular a liposome (Langer, 1990, Science 249:1527-1533; Treat et al., 1989, In: Liposomes in the Therapy of Infectious Disease and Cancer, Lopez-Berestein and Fidler, eds., Liss, New York, 353-365; Lopez-Berestein, ibid., 317-327; see generally ibid.)

In yet another embodiment, the Therapeutic can be delivered via a controlled release system. In one embodiment, a pump may be used (Sefton, 1987, CRC Crit. Ref. Biomed. Eng. 14:201-240; Buchwald et al., 1980, Surgery 88:507-516; Saudek et al., 1989, N. Engl. J. Med. 321:574-579). In another embodiment, polymeric materials can be used (Medical Applications of Controlled Release, Langer and Wise, eds., CRC Press, Boca Raton, Florida, 1974; Controlled Drug Bioavailability, Drug Product Design and Performance, Smolen and Ball, eds., Wiley, New York, 1984; Ranger and Peppas, 1983, Macromol. Sci. Rev. Macromol. Chem. 23:61; Levy et al., 1985, Science 228:190-192; During et al., 1989, Ann. Neurol. 25:351-356; Howard et al., 1989, J. Neurosurg. 71:858-863). In yet another embodiment, a controlled release system can be placed in proximity of the therapeutic target, i.e., the brain, thus requiring only a fraction of the systemic dose (e.g. Goodson, 1984, In: Medical Applications of Controlled Release, supra, Vol. 2, 115-138). Other controlled release systems are discussed in the review by Langer (1990, Science 249:1527-1533).

In order to select an appropriate way of administration, the person skilled in the art will also consider routes of administration which have been selected for other known Anti-Alzheimer-drugs.

For example, Aricept/Donepezil and Cognex/Tacrine (all acetylcholinesterase-inhibitors) are being taken orally, Axura/Memantine (an NMDA-receptor antagonist) has been launched both as tablets/liquid and as an i.v.-solution.

Furthermore, the skilled person in the art will take account the available data with respect to routes of administration of other members of the NSAID-family in clinical trials and other studies investigating their effect on Alzheimer's disease.

In order to select the appropriate dosage, the person skilled in the art will choose a dosage which has been shown to be not toxic in preclinical and/or clinical studies and which can be in accordance with the values given beforehand, or which may deviate from these.

The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 micrograms of active compound per kilogram body weight. Suitable dosage ranges for intranasal administration are generally about 0.01 mg/kg body weight to 1 mg/kg body weight. Effective doses may be extrapolated from dose-response curves derived from in vitro or animal model test systems.

An exemplary animal model is the transgenic mouse strain "Tg2576" containing an APP695-form with the double mutation KM670/671NL. For reference see e.g., patent US5877399 and Hsiao, K et al (1996), Science 274: 99-102 and also Kawarabayahsi T (2001), J. Neurosci 21: 372-81; Frautschy et al (1998), Am J Pathol 152: 307-17, Irizarry et al (1997) J Neuropathol Exp Neurol 56: 965-73; Lehman et al (2003) Neurobiol Aging 24: 645-653.

Substantial data from several studies are available to the skilled person in the art which are instructive to the skilled person to select the appropriate dosage for the chosen therapeutic regimen.

Numerous studies have been published in which the effects of molecules on the γ-secretase activity are described. Exemplary studies are Lim et al, Neurobiol Aging. 2001 Nov-Dec;22(6):983-91, Lim et al, J Neurosci. 2000 Aug 1;20(15):5709-14; Weggen et al. Nature. 2001 Nov 8;414(6860):212-6; Eriksen et al. J Clin Invest. 2003 Aug;112(3):440-9; Yan et al. J Neurosci. 2003 Aug 20;23(20):7504-9; Lanz et al. Abstract. Meeting Soc f. Neurosci, 2003; Friedman et al. Abstract. Meeting Soc f. Neurosci, 2003.

Another aspect of the invention is a preferred process for the identification of a compound according to the present invention comprising the steps of
a. Identifying an aryl acetic acid compound having a chemical structure comprising the substructure of formula (Ib) 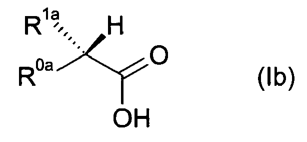 wherein R^{1a} is H or CH₃ and R^{0a} is aryl, as γ-secretase modulator;
b. Preparing a compound having the chemical structure of the compound identified in step a. with a group R¹ and/or R⁰ instead of R^{1a} and/or R^{0a};
c. Testing the compound prepared in step b to determine the inhibition potency against cyclooxygenase activity.

Several γ-secretase modulators are known in the art and may constitute a preferred pool of compounds for the identification of a modulator potentially suitable for a process according to the present invention. A more preferred pool of compounds for the identification includes aryl acetic acid NSAIDs or 2(S)-enantiomers of a 2-arylpropionic acid NSAIDs (2-methylacetic acid NSAIDs).

Potentially suitable NSAIDs are disclosed in US-A 6,160,018.

Other potentially suitable compounds can be identified as R-NSAIDs and may be prepared in step b. as S-enantiomer, e.g. as racemate with subsequent separation of the enantiomers.

In general, every potentially suitable compound can be identified by testing the modulatory effect of that compound towards γ-secretase activity.

Suitable in-vitro and in-vivo assays are available and known to a person skilled in the art (see above).

A modulator of γ-secretase activity (γ-secretase modulator) according to the present invention is preferably a compound showing a Kᵢ of less than 1 mM in a suitable assay.

Preferably, the preparation in step b. is accomplished as mentioned above.

As discussed above the compounds of the invention may have no inhibitory effect towards cyclooxygenase.

A compound according to the present invention having no inhibitory effect towards cyclooxygenase is preferably a compound showing a Kᵢ of more than 1 mM in a suitable assay.

### Examples

### Example 1: Preparation of 2(S)-(3'-trifluoromethyl-biphenyl-4-yl)-3-methyl-butanoic acid

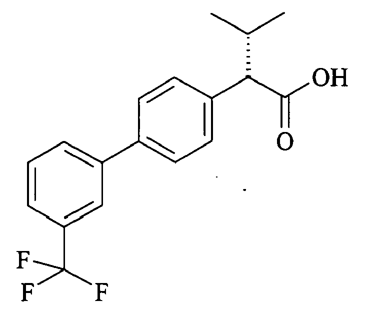

### Preparation of (3'-trifluoromethyl-biphenyl-4-yl)-acetic acid

To a mixture of 3-trifluoromethylphenylboronic acid (0.19g, 1.02mmol), 4-bromophenylacetic acid (0.2g, 0.93mmol) and sodium carbonate (0.3g, 2.8mmol) in water (3ml) is added a catalytic amount of palladium acetate. This mixture is irradiated in a CEM microwave at 120°C for 9 min. The mixture is then filtered through a plug of celite and acidified with HCl (2M) to pH 2. The aqueous layer is then extracted with DCM (x3) and the organics are dried (MgSO₄) and concentrated to afford (3'-trifluoromethyl-biphenyl-4-yl)-acetic acid as a white solid (0.22g, 0.78mmol) in 85% yield. ¹H NMR (CDCl3) δ 10.77 (broad s, 1H); 7.82 (s, 1H), 7.75 (d, 1H), 7.58 (m, 4H), 7.40 (m, 2H), 3.73 (s, 2H); LCMS method (4), MH- (279), RT = 4.0min.

### Preparation of (3'-trifluoromethyl-biphenyl-4-yl)-acetic acid methyl ester

To a solution of 3'-trifluoromethyl-biphenyl-4-yl)-acetic acid (0.5g, 2.54mmol) in methanol (14ml) is added a catalytic amount of sulphuric acid. The reaction mixture is stirred overnight and then concentrated. The oil is diluted with EtOAc (45ml), quenched by the addition of sat. NaHCO₃ then washed with brine (x2) and dried (MgSO₄). The organics were concentrated and the yellow oil was purified by flash chromatography (0-20% EtOAc: petroleum ether) to afford (3'-trifluoromethyl-biphenyl-4-yl)-acetic acid methyl ester as a colourless oil (0.55g, 2.43mmol) in 96% yield. ¹H NMR (CDCl3) δ 7.82 (s, 1H), 7.76 (d, 1H), 7.58 (m, 4H), 7.40 (d, 1H), 3.73 (s, 3H), 3.70 (s, 2H); LCMS method (3), RT = 4.5min.

### Preparation of 2(S)-(3'-trifluoromethyl-biphenyl-4-yl)-3-methyl-butanoic acid

A solution of LDA in hexanes (1.89mL, 3.4mmol, 1.8M) is added dropwise to a solution of (3'-Trifluoromethyl-biphenyl-4-yl)-acetic acid methyl ester (0.5g, 1.7mmol) in THF (8ml) at -78°C. After 30 min 2-bromopropane (5.1mmol) in THF (2ml) is added and the mixture slowly allowed to warm to room temperature. Upon completion of reaction (3h) the mixture is quenched by the addition of sat. NH₄Cl. This is then extracted with DCM (x3), washed with HCl (0.5N, 15mL) and the organics are dried (MgSO₄) and concentrated *in vacuo* to afford a brown oil which is dissolved in THF (11ml) and water (11ml) and cooled to 0°C. Lithium hydroxide (0.18g, 7.46mmol) is dissolved in THF (2ml) and added dropwise to the solution. The mixture is stirred for a further 1h then allowed to warm to room temperature for 16h. The mixture is acidified by addition of HCl (1M) and extracted with DCM. The organics were washed with water dried (MgSO₄) and concentrated to afford 2(R, S)-(3'-trifluoromethyl-biphenyl-4-yl)-3-methyl-butanoic acid. ¹H NMR (CDCl3) δ 7.81 (s, 1H), 7.74 (d, 1H), 7.55 (m, 4H), 7.44 (m, 2H), 3.22 (d, 1H), 2.38 (m, 1H), 1.11 (d, 3H), 0.76 (d, 3H); LCMS method (4), (M-H-) 320.9, RT = 4.5min. The S enantiomer is obtained by HPLC using a chiral stationary phase.

### Example 2: Screening of the compounds of the invention for γ-secretase-modulating activity

Screening is carried out using SKN neuroblastoma cells carrying the APP-"swedish mutation" (point mutation at 695 of APP) grown in DMEM/NUT-mix F12 (HAM) provided by Gibco (cat no. 31330-38) containing 5% Serum/Fe supplemented with 1% non-essential amino acids, 100 U/ml Pen/Strep.

Cells are grown to near confluency.

The screening is performed using the assay as described in Citron et al. (1997) Nature Medicine. Vol 3: 67-72.

### IC50-values of selected compounds of the invention on the γ-secretase activity.

The title compound of example 1 shows an IC50 value <100µM.

### Example 3: Determination of the effect the compounds according to the invention on cyclooxygenase-1 (Cox-1)

Inhibition of Cox-1 is determined using the Colorimetric Cox inhibitor screening assay provided by Cayman Chemical Company, Ann Arbor, MI, USA. (Cat. No. 760111) according to manufacturer's instructions.

The title compound of example 1 shows no inhibitory effect against Cox-1 activity.

## Claims

1. An aryl acetic acid γ-secretase modulator compound having a chemical structure comprising the substructure of formula (Ia) 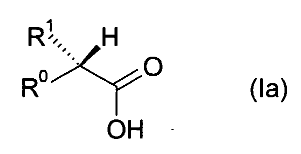 or a pharmaceutically acceptable salt or ester thereof, wherein
R⁰ is aryl and R¹ is other than H, COOH or R°, provided that at least one of R⁰, R¹ is selected to prevent inhibition of cyclooxygenase activity by the compound.

2. A compound according to claim 1 having the formula (I) 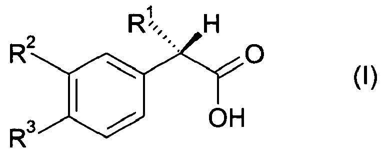 or a pharmaceutically acceptable salt or ester thereof, wherein
R¹ is other than H, COOH or 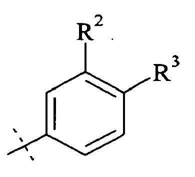 and at least one of R¹, R², R³ is selected to prevent inhibition of cyclooxygenase activity by the compound of formula (I).

3. A compound according to claim 1 or 2, wherein R², R³ are independently selected from the group consisting of H; halogen; C₁₋₄ alkyl; C₂₋₄ alkenyl; O-C₁₋₄-alkyl; and R⁴-Q; wherein each C₁₋₄ alkyl and C₂₋₄ alkenyl is optionally substituted with one or more halogen, which are the same or different; provided that at least one of R², R³ includes a cyclic moiety;
Optionally, R², R³ form together with the carbon atoms to which they are attached to a ring R⁵;
Q is a covalent bond; -C(O)-; -O-; -CH₂-O-; -O-CH₂-; -NH-; -CH₂-NH-; -NH-CH₂-
R⁴, R⁵ are independently selected from the group consisting of phenyl; heterocyclyl and heterobicyclyl and optionally substituted with one or more R⁶ independently selected from the group consisting of halogen; C₁₋₄ alkyl; O-C₂₋₄ alkenyl; phenyl; heterocyclyl; heterobicyclyl; OH; and oxo (=O), where the ring is at least partially saturated; wherein each C₁₋₄ alkyl; C₂₋₄ alkenyl; phenyl; heterocyclyl and heterobicyclyl is optionally substituted with one or more halogen, which are the same or different.

4. A compound according to any of the claims 1 to 3, wherein R¹ is C₂₋₄ alkyl; C₂₋₄ alkenyl; O-C₁₋₄-alkyl; and R^{4a}-Q^{a}; wherein each C₂₋₄ alkyl and C₂₋₄ alkenyl is optionally substituted with one or more halogen, which are the same or different and wherein Q^{a} is a covalent bond; -C(O)-; -CH₂-O- or -O-CH₂-; and R^{4a} is phenyl; heterocyclyl or heterobicyclyl and optionally substituted with one or more R^{6a} independently selected from the group consisting of halogen; C₁₋₄ alkyl; O-C₂₋₄ alkenyl; OH; oxo (=O), where the ring is at least partially saturated; wherein each C₁₋₄ alkyl and C₂₋₄ alkenyl is optionally substituted with one or more halogen, which are the same or different.

5. A compound according to any of the claims 1 to 4, wherein one of R², R³ is H or F and the other is R⁴-Q.

6. A compound according to any of the claims 1 to 5, wherein R⁴ is phenyl; thiophenyl; 2,5-dihydropyrrolyl; or isoindolin-2one-3-yl.

7. A compound according to any of the claims 1 to 4, wherein R⁵ is phenyl; indolyl; or oxazolyl.

8. A compound according to any of the claims 1 to 7, wherein R⁶ is H; CH₃; CF₃; OCF₃; F; Cl; Br or I.

9. A pharmaceutical composition comprising a compound or a pharmaceutically acceptable salt thereof according to any of the claims 1 to 8 together with a pharmaceutically acceptable carrier.

10. A pharmaceutical composition according to claim 9, comprising one or more additional compounds or pharmaceutically acceptable salts thereof selected from the group consisting of compounds according to any of the claims 1 to 6 and not being the first compound; other pharmaceutically active compounds suitable to treat or prevent Alzheimer's disease; and other γ-secretase modulators.

11. A compound or a pharmaceutically acceptable salt thereof according to any of claims 1 to 8 for use as a medicament.

12. Use of a compound or a pharmaceutically acceptable salt thereof according to any of claims 1 to 8 for the manufacture of a medicament for the treatment or prophylaxis of at least one of the following diseases:
a. Alzheimer's disease;
b. One or more diseases associated with the modulation of γ-secretase activity;
c. One or more diseases associated with an elevated level of Aβ42-production.

13. Use of a compound according to any of the claims 1 to 8 for the modulation of y-secretase activity.

14. Process for the identification of a compound according to any of the claims 1 to 8 comprising the steps of
a Identifying an aryl acetic acid compound having a chemical structure comprising the substructure of formula (Ib) 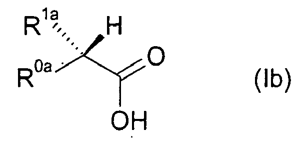 wherein R^{1a} is H or CH₃ and R^{0a} is aryl, as γ-secretase modulator;
b Preparing a compound having the chemical structure of the compound identified in step a. with a group R¹ and/or R⁰ instead of R^{1a} and/or R^{0a};
c Testing the compound prepared in step b to determine the inhibition potency against cyclooxygenase activity.

15. Process according to claim 14, wherein R^{1a} is modified.

16. Process according to claim 14 or 15, wherein the aryl acetic compound is an aryl acetic acid NSAID or the 2(S)-enantiomer of a 2-arylpropionic acid NSAID (2-methylacetic acid NSAID).
